# EUROPEAN PATENT APPLICATION

(11) **EP 4 112 090 A1**
(43) Date of publication of application: **04.01.2023**
(21) Application number: 20921069.9
(22) Date of filing: 13.03.2020
(51) Int. Cl.: A61L 27/06, A61L 27/30, A61L 27/54, A61L 27/56, C22C 14/00, C25D 11/02, C25D 11/26, A61L 27/58

(54) **ANTIBACTERIAL THREE-DIMENSIONAL POROUS BONE IMPLANT MATERIAL AND PREPARATION METHOD THEREFOR**

(30) Priority: 25.02.2020 CN 202010116870; 25.02.2020 CN 202010116858
(71) Applicant: Beijing AK Medical Co., Ltd., 102200, Beijing (CN)
(72) Inventor: WEI, Chongbin, Beijing 102200 (CN)
(74) Representative: Biggi, Cristina
(86) International application number: PCT/CN2020/079275
(87) International publication number: WO 2021/168917

(57) **Abstract**

An antibacterial three-dimensional porous bone implant material. The antibacterial three-dimensional porous bone implant material comprises: a three-dimensional porous bone implant material; and an in-situ growth film layer in-situ growing on the surface of the three-dimensional porous bone implant material, wherein the in-situ growth film layer comprises a functional substance and an antibacterial substance, and the antibacterial substance comprises any one or more of zinc ions, copper ions or silver ions. The in-situ growth film layer has an antibacterial effect. The macro pore size and the micro pore size of the antibacterial three-dimensional porous bone implant material coexist, micro pores in a micro-arc oxidation film layer on a porous wall can provide anchoring points for bone growth, and the specific surface area of the implant material is effectively increased, and thus, the implant material in the early stage of implantation can have an antibacterial function and the biologically active functions of bone growth and bone induction.

## Description

### Technical Field

The present disclosure relates to the field of medical devices, and in particular, to an antibacterial three-dimensional porous bone implant material and a preparation method therefor.

### Background

With the improvement of medical technology and the development of economic level, various implants, such as titanium and titanium alloy, used for orthopedic replacement, orthopedic, fixation and other functions are increasingly applied to surgery. In recent years, porous bone implant materials, such as additive manufacturing titanium alloys, have also been used clinically as bone implant materials for commercial applications, achieving the purpose of relieving patients' pain and restoring function.

Placing an implant material in a human body is a traumatic surgery, and postoperative complications are also prone to occur, especially the infection caused by implants has become the most serious and even catastrophic complications of its use, especially in open fracture and joint revision, which often requires multiple times of surgical debridement and removal of an internal fixation or a prosthesis, resulting in related problems such as surgical failure, increased costs, and increased disability of patients.

There are many factors causing infection, one of which is the infection caused by the implant materials. The initial process of the infection related to the implant materials is bacterial adhesion on the surface of prosthesis. In order to reduce the occurrence of the infection related to implant materials, an important measure is to study the antibacterial performance of the surface of the implant materials. The literature *"The effect of applied voltages on the structure, apatite-inducing ability and antibacterial ability of micro arc oxidation coating formed on titanium surface"* studies the study of electrical parameters on the antibacterial properties of two-dimensional pure titanium surfaces. The patent application with publication No. CN107661544A discloses that gentamicin and the like are loaded on a 3D printing titanium alloy material to achieve an antibacterial effect. However, at present, there are no reports on the technology of modifying the surface of porous bone implant material by electrochemical method to impart a bioactive film layer on the surface and impart antibacterial performance, and achieving industrial production. The applicant of the present disclosure discloses, in the patent of invention with the patent No. ZL201710118388.9, a surface-modified porous metal implant and a preparation method therefor. The preparation method can achieve a uniform bioactive film layer on a porous surface on an industrial scale, but cannot impart antibacterial performance to the bioactive film layer.

At present, the bioactivity and antibacterial function are achieved on the surface of bone implant material by means of the micro-arc oxidation technology, mostly on a two-dimensional surface of a solid material with a consistent structure. A three-dimensional porous structure has an increased resistance per unit volume when micro-arc oxidation is performed, and particularly when a porous material has a relatively large volume, it is difficult to arc the material and form an oxide film by means of traditional methods. The porous structure is more likely to cause uneven current distribution on the material, and is easy to cause uneven distribution of the film layer and uneven distribution of bioactive ions in the film layer. In particular, antibacterial substances, such as silver ions, copper ions and zinc ions, are difficult to achieve adhesion on the porous material and uniform distribution in the film layer in the current micro-arc oxidation process, and thus the ideal antibacterial effect cannot be achieved.

### Summary

A main objective of the present invention is to provide an antibacterial three-dimensional porous bone implant material and a preparation method therefor, so as to solve the problem in the prior art that the three-dimensional porous bone implant material has a poor antibacterial performance.

In order to achieve the described objective, according to one aspect of the present invention, provided is an antibacterial three-dimensional porous bone implant material, the antibacterial three-dimensional porous bone implant material comprises: a three-dimensional porous bone implant material; and an in-situ growth film layer in-situ growing on the surface of the three-dimensional porous bone implant material, the in-situ growth film layer comprises a functional substance and an antibacterial substance, and the antibacterial substance comprises any one or more of zinc ions, copper ions or silver ions.

Further, the in-situ growth film layer has a thickness of 0.1 to 20 µm, preferably 2 to 7 µm.

Further, the surface of the in-situ growth film layer comprises micropores with a pore diameter of 0.1 to 5 µm, and preferably the in-situ growth film layer has a porosity of 2 to 30%.

Further, in the in-situ growth film layer, the content of the zinc ions is 0.5 to 3 at.%, and/or the content of the copper ions is 0.5 to 3 at.%, and/or the content of the silver ions is 0.01 to 2 at.%.

Further, the surface on which the three-dimensional porous bone implant material is in contact with the host bone tissue is porous structure titanium or porous titanium alloy.

Further, the three-hole structure titanium is one of pure titanium, Ti-6AI-4V, Ti-6AI-17Nb, Ti-13Nb-13Zr or Ti-5Zr-3Mo-15Nb, and the porous titanium alloy is one of Ti-6AI-4V, Ti-6AI-17Nb, Ti-13Nb-13Zr or Ti-5Zr-3Mo-15Nb.

Further, the three-dimensional porous bone implant material is in a fully porous structure or a mixed structure of porous and solid structures.

Further, the three-dimensional porous bone implant material has a porosity of 40 to 90% and a pore diameter of 200 to 1500 µm.

Further, the porous structure of the three-dimensional porous bone implant material is one or more of a random pore structure, a cubic structure, a hexagonal prism structure, a diamond structure, a rhombic dodecahedron structure, a truncated octahedron structure, a titanium bead sintering structure, and a titanium wire sintering structure.

Further, the three-dimensional porous bone implant material is a columnar structure, a columnar-like structure, a plate structure, a hemisphere structure, a block structure, a spherical particle shape, a conical or a sleeve shape.

Further, the three-dimensional porous bone implant material is an interbody fusion cage, and the surface structure of the interbody fusion cage is a porous structure.

Further, the three-dimensional porous bone implant material is an artificial vertebral body, and the surface of the artificial vertebral body is a porous structure.

Further, the three-dimensional porous bone implant material is an acetabular cup or an acetabular augment, and the surface structure of the acetabular cup or the acetabular augment is one or more of a porous structure, a titanium bead, a titanium powder, and a titanium wire.

Further, the three-dimensional porous bone implant material is a femoral stem, and the proximal surface structure of the femur of the femoral stem is a porous structure.

According to another aspect of the present invention, provided is a preparation method for an antibacterial three-dimensional porous bone implant material, comprising: performing surface activation treatment on the three-dimensional porous bone implant material so as to obtain a pretreated material; performing micro-arc oxidation treatment on the pretreated material so as to obtain an in-situ growth film layer having antibacterial ions formed on the surface of the pretreated material, wherein an electrolyte used in the micro-arc oxidation treatment comprises antibacterial ions and a functional substance, and the antibacterial ions are selected from any one or more of zinc ions, copper ions and silver ions; and performing hydrothermal treatment on the in-situ growth film layer so as to obtain the antibacterial three-dimensional porous bone implant material.

Further, the process of the surface activation treatment comprises: pickling the three-dimensional porous bone implant material, wherein the pickling solution used in the pickling comprises hydrofluoric acid, nitric acid and water, and is metered with the hydrofluoric acid having a concentration of 40%, the nitric acid having a concentration of 68% and the water, the volume ratio of the hydrofluoric acid, the nitric acid and the water is (5-7): (10-15): (80-85), and the pickling time is preferably 2-30 minutes; after the pickling is finished, washing the three-dimensional porous bone implant material with deionized water, so as to obtain the pretreated material; and preferably, the washing comprises first ultrasonic washing for 5 to 15 minutes, and after the ultrasonic washing is finished, spraying, washing and then drying the three-dimensional porous bone implant material.

Further, in the electrolyte, the zinc ions are derived from zinc acetate or zinc sulfate, and preferably the concentration of the zinc acetate is 1 to 35 g/L or the concentration of the zinc sulfate is 1 to 20 g/L; the copper ions are derived from copper acetate or copper sulfate, and preferably the concentration of the copper acetate is 1 to 20 g/L or the concentration of the copper sulfate is 1 to 20 g/L; and the silver ions are derived from silver acetate or silver nitrate, and preferably the concentration of the silver acetate is 0.01 to 40 g/L or the concentration of the silver nitrate is 0.01 to 35 g/L.

Further, the oxidation voltage of the micro-arc oxidation treatment is between 400 and 1200 V, the output pulse frequency of the power source is between 600 and 1500 Hz, the peak current is set to be 300 and 800 A, the oxidation time is between 5 and 60 min, and the temperature of the electrolyte is controlled to be lower than 10°C in the process of the micro-arc oxidation treatment.

Further, the process of hydrothermal treatment comprises: step A, washing the three-dimensional porous bone implant material having the in-situ growth film with deionized water, so as to obtain a washed film; step B, placing the three-dimensional porous bone implant material having the washed film in an alkaline solution and treating the material in an environment of 120-200°C and 0.5-15 MPa so as to obtain a hydrothermal treatment film, wherein the alkaline solution is preferably a sodium hydroxide aqueous solution or a potassium hydroxide aqueous solution having a pH value of 8-12, and the treatment time is preferably 8-32 hours; and step C, washing the three-dimensional porous bone implant material having the hydrothermal treatment film with deionized water so as to obtain the antibacterial three-dimensional porous bone implant material; and preferably, the washing in the step A and the step C adopts ultrasonic vibration treatment.

Further, the surface on which the three-dimensional porous bone implant material is in contact with the host bone tissue is porous structure titanium or porous titanium alloy; preferably, the preparation method further comprises a step of preparing the porous bone implant material by using one or more of a powder metallurgy method, a foaming method, a fiber sintering method, a plasma spraying method, a laser drilling method, laser stereoforming, selective laser sintering/melting, and electron beam melting technologies.

Further, the porous structure titanium is one of pure titanium, Ti-6AI-4V, Ti-6AI-17Nb, Ti-13Nb-13Zr or Ti-5Zr-3Mo-15Nb; the porous titanium alloy is one of Ti-6AI-4V, Ti-6AI-17Nb, Ti-13Nb-13Zr or Ti-5Zr-3Mo-15Nb; preferably, the three-dimensional porous bone implant material having a structure pore which is in a columnar structure, a columnar-like structure, a plate structure, a hemisphere structure, a block structure, a spherical particle shape, a conical or a sleeve shape; preferably, the three-dimensional porous bone implant material is in a fully porous structure, or a mixed structure of porous and solid structures; more preferably, the three-dimensional porous bone implant material has a porosity of 40 to 90% and a pore diameter of 200 to 1500 µm; further preferably, the porous structure of the three-dimensional porous bone implant material is in one or more of a random pore structure, a cubic structure, a hexagonal prism structure, a diamond structure, a rhombic dodecahedron structure, a truncated octahedron structure, a titanium bead sintering structure, and a titanium wire sintering structure.

Further, the in-situ growth film layer has a thickness of 0.1 to 20 µm; preferably, the surface of the in-situ growth film layer comprises micropores with a pore diameter of 0.1 to 5 µm; and preferably the in-situ growth film layer has a porosity of 2 to 30%.

Further, the three-dimensional porous bone implant material is an acetabular cup or an acetabular augment, and the surface structure of the acetabular cup or the acetabular augment is one or more of a porous structure, a titanium bead, a titanium powder, and a titanium wire.

Further, the three-dimensional porous bone implant material is an interbody fusion cage, and the surface structure of the interbody fusion cage is a porous structure.

Further, the three-dimensional porous bone implant material is an artificial vertebral body, and the surface structure of the artificial vertebral body is a porous structure.

Further, the three-dimensional porous bone implant material is a femoral stem, and the proximal surface structure of the femur of the femoral stem is a porous structure.

By applying the technical solution of the present invention, as the in-situ growth film of the present disclosure comprises an antibacterial substance, the in-situ growth film has an antibacterial effect. Specifically, the existence of the above-mentioned in-situ growth film layer not only can improve the antibacterial and bacteriostatic properties and bioactivity of the three-dimensional porous implant material, but also can avoid the corrosion of the surface of the material and the risk of the metal ions entering human tissues caused by the long-term existence of the implant material in the human body. It is verified by experiments that the in-situ growth film layer can effectively inhibit bacteria, reduce the aggregation of bacteria on an implanted material, effectively reduce the risk of infection caused by implantation of a prosthesis, improve the safety and effectiveness of the implanted material in use, especially have a significant inhibitory effect on Staphylococcus aureus, Escherichia coli and the like, can impart a single antibacterial substance to a three-dimensional porous implanted material according to needs, and can also provide multiple antibacterial substances at the same time, so as to achieve an ideal antibacterial and bacteriostatic effect. The above-mentioned antibacterial three-dimensional porous bone implant material has a coexistence of a macro pore size and a micro pore size, micro pores in a micro-arc oxidation film layer on a porous wall can provide anchoring points for bone growth, and effectively increase the specific surface area of the implant material, and thus the implant material in the early stage of implantation can have an antibacterial function and the biologically active functions of bone growth and bone induction, and has good prospect of applications on a high-end orthopedic implant prosthesis.

In the present disclosure, before micro-arc oxidation treatment, performing surface activation treatment on a three-dimensional porous bone implant material improves the arcing ability on the surface and hole thereof, and further facilitates the generation of micro-arc oxidation; free antibacterial ions are doped into an electrolyte, and then the free antibacterial ions along with a functional substance in situ grow on the surface and hole of the three-dimensional porous bone implant material under the action of the micro-arc oxidation, thereby forming an in-situ growth film layer having antibacterial ions; in the micro-arc oxidation treatment, as an anode has a strong repulsion effect on the cations of the antibacterial ions, the elements mainly enter the in-situ growth film layer by means of a diffusion effect, and therefore, both the content and stability of antibacterial ions in the surface layer thereof are lower than the content and stability of antibacterial ions in the inner layer. In order to improve the distribution uniformity and stability of the antibacterial ions in the in-situ growth film layer, the in-situ growth film layer is further subjected to hydrothermal treatment after the micro-arc oxidation; and during the hydrothermal treatment, calcium and phosphorus in the in-situ growth film layer form a hydroxyapatite structure, while antibacterial ions also migrates in the film, thereby obtaining a three-dimensional porous bone implant material comprising the antibacterial ions and stable bioactive elements.

### Brief Description of the Drawings

The accompanying drawings, which form a part of the present application, are used to provide a further understanding of the present invention. The schematic examples of the present invention and the description thereof are used to explain the present invention, and do not form improper limitation of the present invention. In the drawings:
Fig. 1 shows a scanning electron microscope image of an in-situ growth film layer according to example 1 of the present invention, with a magnification of 5000 times; and
Fig. 2 shows a scanning electron microscope image of an in-situ growth film layer according to example 1 of the present invention, with a magnification of 500 times.

### Detailed Description of the Embodiments

It should be noted that the examples of the present disclosure and the characteristics in the examples can be combined with each other under the condition of no conflicts. The present disclosure will be described below with reference to the drawings and examples in detail.

As analyzed in the background art of the present disclosure, the in-situ growth film formed by the micro-arc oxidation process of the prior art has no antibacterial properties because it does not have antibacterial substances. In order to solve the problem, some embodiments of the present disclosure provide an antibacterial three-dimensional porous bone implant material and a preparation method therefor.

In a typical embodiment of the present disclosure, provided is an antibacterial three-dimensional porous bone implant material, the antibacterial three-dimensional porous bone implant material comprises a three-dimensional porous bone implant material and an in-situ growth film layer in-situ growing on the surface of the three-dimensional porous bone implant material, wherein the in-situ growth film layer comprises a functional substance and an antibacterial substance, and the antibacterial substance comprises any one or more of zinc ions, copper ions or silver ions.

As the in-situ growth film of the present disclosure comprises an antibacterial substance, the in-situ growth film has an antibacterial effect. Specifically, the existence of the above-mentioned in-situ growth film layer not only can improve the antibacterial and bacteriostatic properties and biological activity of the three-dimensional porous implant material, but also can avoid the corrosion of the surface of the material and the risk of the metal ions entering human tissues caused by the long-term existence of the implant material in the human body. It is verified by experiments that the in-situ growth film layer can effectively inhibit bacteria, reduce the aggregation of bacteria on an implanted material, effectively reduce the risk of infection caused by implantation of a prosthesis, improve the safety and effectiveness of the implanted material in use, especially have a significant inhibitory effect on Staphylococcus aureus, Escherichia coli and the like, can impart a single antibacterial substance to a three-dimensional porous implanted material according to needs, and can also provide multiple antibacterial substances at the same time, so as to achieve an ideal antibacterial and bacteriostatic effect. The above-mentioned antibacterial three-dimensional porous bone implant material has a coexistence of a macro pore size and a micro pore size, micro pores in a micro-arc oxidation film layer on a porous wall can provide anchoring points for bone growth, and effectively increase the specific surface area of the implant material, and thus the implant material in the early stage of implantation can have an antibacterial function and the biologically active functions of bone growth and bone induction, and has good prospect of applications on a high-end orthopedic implant prosthesis.

The described in situ growth film is formed by a micro-arc oxidation process. In a typical embodiment of the present disclosure, provided is a preparation method for an antibacterial three-dimensional porous bone implant material, the method comprises: performing surface activation treatment on the three-dimensional porous bone implant material, so as to obtain a pretreated material; performing micro-arc oxidation treatment on the pre-treated material, so as to obtain an in-situ growth film layer having antibacterial ions formed on the surface of the pre-treated material, wherein an electrolyte used in the micro-arc oxidation treatment comprises antibacterial ions and a functional substance, and the antibacterial ions are selected from any one or more of zinc ions, copper ions and silver ions; and performing hydrothermal treatment on the in-situ growth film layer, so as to obtain the antibacterial three-dimensional porous bone implant material.

In the present disclosure, before micro-arc oxidation treatment, performing surface activation treatment on a three-dimensional porous bone implant material improves the arcing ability on the surface and hole thereof, and further facilitates the generation of micro-arc oxidation; free antibacterial ions are doped into an electrolyte, and then the free antibacterial ions along with a functional substance in situ grow on the surface and hole of the three-dimensional porous bone implant material under the action of the micro-arc oxidation, thereby forming an in-situ growth film layer having antibacterial ions; in the micro-arc oxidation treatment, as an anode has a strong repulsion effect on the cations of the antibacterial ions, the elements mainly enter the in-situ growth film layer by means of a diffusion effect, and therefore, both the content and stability of antibacterial ions in the surface layer thereof are lower than the content and stability of antibacterial ions in the inner layer. In order to improve the distribution uniformity and stability of the antibacterial ions in the in-situ growth film layer, the in-situ growth film layer is further subjected to hydrothermal treatment after the micro-arc oxidation; and during the hydrothermal treatment, calcium and phosphorus in the in-situ growth film layer form a hydroxyapatite structure, while antibacterial ions also migrates in the film, thereby obtaining a three-dimensional porous bone implant material having a stable structure.

The antibacterial three-dimensional porous bone implant material formed by the preparation method comprises a three-dimensional porous bone implant material and an in situ growth film layer, wherein the in situ growth film layer comprises antibacterial ions, and the antibacterial ions comprise any one or more of the zinc ions, the copper ions or the silver ions.

The surface activation treatment is mainly to remove impurities and an oxide layer from the surface of the three-dimensional porous bone implant material. In order to optimize the surface activation treatment effect, the process of surface activation treatment preferably comprises: pickling the three-dimensional porous bone implant material, wherein the pickling solution used in the pickling comprises hydrofluoric acid, nitric acid and water, and is metered with the hydrofluoric acid having a concentration of 40%, the nitric acid having a concentration of 68% and the water, the volume ratio of the hydrofluoric acid, the nitric acid and the water is (5-7): (10-15): (80-85), and the pickling time is preferably 2-30 minutes; after the pickling is finished, washing the three-dimensional porous bone implant material with deionized water, so as to obtain the pretreated material; and preferably, the washing comprises first ultrasonic washing for 5 to 15 minutes, and after the ultrasonic washing is finished, spraying, washing and then drying the three-dimensional porous bone implant material. The pickling solution is used to strongly corrode the surface of the three-dimensional porous bone implant material for removing impurities and oxides from its surface, thereby providing more attachment surfaces for antibacterial ions. The washing process after pickling further removes the residues from the surface of the three-dimensional porous bone implant material, facilitating the oxidation.

As an electrolyte for micro-arc oxidation is generally an alkaline solution, in order to increase the dissociation ability of an antibacterial ion in the electrolyte as for as possible, the zinc ions in the electrolyte are preferably derived from zinc acetate or zinc sulfate; and in addition, in order to increase the proportion of the zinc ions growing in situ as for as possible, the concentration of the zinc acetate is preferably 1 to 35 g/L or the concentration of the zinc sulfate is preferably 1 to 20 g/L. The copper ions are derived from copper acetate or copper sulfate; in addition, in order to increase the proportion of the copper ions growing in situ as for as possible, the concentration of the copper acetate is 1 to 20 g/L or the concentration of the copper sulfate is 1 to 20 g/L; the silver ions are derived from silver acetate or silver nitrate; in addition, in order to increase the proportion of the silver ions growing in situ as for as possible, the concentration of the silver acetate is preferably 0.01 to 40 g/L or the concentration of the silver nitrate is preferably 0.01 to 35 g/L.

In addition, on the basis of existing micro-arc oxidation devices, in the present disclosure, in order to increase the loading amount of antibacterial elements, the functional elements in the electrolyte preferably comprise calcium and phosphorus, the concentration of the calcium element in the electrolyte is recorded as m mol/L, and the concentration of the phosphorus element is recorded as n mol/L; when 0.01≤m<0.2, (0.1m+0.025)≤n<0.05, and when 0.2≤m≤0.6, 0.075≤n≤m/0.875, preferably 0.4<m≤0.6, and 0.25≤n≤m/0.875, preferably, the calcium source is selected from one or more of calcium acetate, calcium chloride, calcium dihydrogen phosphate, calcium glycerophosphate, calcium citrate, calcium lactate and calcium oxide; more preferably, the phosphorus source is selected from one or more of sodium glycerophosphate, sodium dihydrogen phosphate, disodium hydrogen phosphate, sodium hexametaphosphate, and sodium polyphosphat; preferably, EDTA-2Na is added to the electrolyte as a complexing agent, and the concentration of the EDTA in the electrolyte is recorded as x mol/L, when 0.01≤m<0.2, 2m≤x<5.2m, and when 0.2≤m≤0.6, 5.2m≤x≤7m.

On the basis of achieving the efficient in situ growth of the functional elements calcium and phosphorus, in order to further improve the in situ growth capacity of the antibacterial elements, sodium hydroxide or potassium hydroxide is preferably added into the described electrolyte to adjust the pH value of the electrolyte to be between 11 and 14, and more preferably between 12 and 14.

In an embodiment of the present disclosure, the oxidation voltage of the micro-arc oxidation treatment is between 400 and 1200 V, the output pulse frequency of the power source is between 600 and 1500 Hz, the peak current is set to be 300 and 800 A, the oxidation time is between 5 and 60 min, and the temperature of the electrolyte is preferably controlled to be lower than 10°C in the process of the micro-arc oxidation treatment. Under the described process conditions of the micro-arc oxidation treatment, the thickness of the formed in-situ growth film is easy to control, and the element ratio therein is also relatively reasonable.

The effect of the hydrothermal treatment in the present disclosure is as described above, in order to improve the mobility of the antibacterial elements, preferably, the process of hydrothermal treatment comprises: step A, washing the three-dimensional porous bone implant material having the in-situ growth film with deionized water, so as to obtain a washed film; step B, placing the three-dimensional porous bone implant material having the washed film in an alkaline solution and treating the material in an environment of 120-200°C and 0.5-15 MPa, so as to obtain a hydrothermal treatment film, wherein the alkaline solution is preferably a sodium hydroxide aqueous solution or a potassium hydroxide aqueous solution having a pH value of 8-12, and the treatment time is preferably 2-32 hours; and step C, washing the three-dimensional porous bone implant material having the hydrothermal treatment film with deionized water, so as to obtain the antibacterial three-dimensional porous bone implant material; and preferably, the washing in the step A and the step C adopts ultrasonic vibration treatment.

In order to better meet the needs of the working environment of micro-arc oxidation and the standard requirements for medical devices, it is preferred that the above-mentioned materials for connecting the cathode with the electrolytic tank are all stainless steel.

In addition, it is preferred that the surface on which the three-dimensional porous bone implant material is in contact with the host bone tissue is porous structure titanium or porous titanium alloy; preferably, the preparation method further comprises a step of preparing the porous bone implant material by using one or more of a powder metallurgy method, a foaming method, a fiber sintering method, a plasma spraying method, a laser drilling method, laser stereoforming, selective laser sintering/melting, and electron beam melting technologies.

When the porous structure titanium is used, the porous structure titanium used in the present disclosure can be one of pure titanium, Ti-6AI-4V, Ti-6AI-17Nb, Ti-13Nb-13Zr or Ti-5Zr-3Mo-15Nb; when the porous titanium alloy is used, the porous titanium alloy used in the present disclosure can be one of Ti-6AI-4V, Ti-6AI-17Nb, Ti-13Nb-13Zr or Ti-5Zr-3Mo-15Nb. The above preparation method of the present disclosure is applicable to a three-dimensional porous bone implant material of various shapes. Preferably, the three-dimensional porous bone implant material having a structure pore which is in a columnar structure, a columnar-like structure, a plate structure, a hemisphere structure, a block structure, a spherical particle shape, a conical or a sleeve shape. In addition, micro-arc oxidation mainly forms an in-situ growth film on the surface of the three-dimensional porous bone implant material, therefore, the three-dimensional porous structure on the surface of the material can be used in the present disclosure, preferably, the three-dimensional porous bone implant material is in a fully porous structure, or a mixed structure of porous and solid structures. More preferably, the three-dimensional porous bone implant material has a porosity of 40 to 90% and a pore diameter of 200 to 1500 µm, further preferably, the porous structure of the three-dimensional porous bone implant material is in one or more of a random pore structure, a cubic structure, a hexagonal prism structure, a diamond structure, a rhombic dodecahedron structure, a truncated octahedron structure, a titanium bead sintering structure, and a titanium wire sintering structure.

The thickness of the in-situ growth film layer can be adjusted by controlling conditions such as the time and process of the micro-arc oxidation. Preferably, the in-situ growth film layer has a thickness of 0.1 to 20 µm, preferably 2 to 7 µm.

In addition, the surface of the above in-situ growth film layer preferably comprises micropores with a pore diameter of 0.1 to 5 µm, and preferably the in-situ growth film layer has a porosity of 2 to 30%. The described pore diameter and porosity can also be achieved by controlling the process condition of the micro-arc oxidation. A person skilled in the art can obtain the realization conditions of the corresponding pore diameter and porosity by means of experiments within the described range of conditions in the present disclosure, which is not redundantly described herein.

On the basis of ensuring that the in-situ growth film layer has an appropriate biological activity, in order to improve the antibacterial performance thereof, preferably, in the described in-situ growth film layer, the content of zinc ions is 0.5 to 3 at.%, and/or the content of copper ions is 0.5 to 3 at.%, and/or the content of silver ions is 0.01 to 2 at.%.

The three-dimensional porous bone implant material used in the present disclosure can be a three-dimensional porous bone implant material commonly used in the prior art, preferably, the surface on which the three-dimensional porous bone implant material is in contact with the host bone tissue is porous structure titanium or porous titanium alloy, preferably, the porous titanium alloy is one of Ti-6AI-4V, Ti-6AI-17Nb, Ti-13Nb-13Zr or Ti-5Zr-3Mo-15Nb.

In an embodiment of the present disclosure, the described three-dimensional porous bone implant material is in a fully porous structure or a mixed structure of porous and a solid structures; more preferably, the porosity of the three-dimensional porous bone implant material is 40 to 90% and the pore size is 200 to 1500 µm to increase the growth area of the in-situ growth film layer; further preferably, the porous structure of the three-dimensional porous bone implant material is in one or more of a random pore structure, a cubic structure, a hexagonal prism structure, a diamond structure, a rhombic dodecahedron structure, a truncated octahedron structure, a titanium bead sintering structure, and a titanium wire sintering structure.

The three-dimensional porous bone implant material of the present disclosure can be of various shapes, according to the current shape requirements of the bone implant material, preferably, the above-mentioned three-dimensional porous bone implant material is a columnar structure, a columnar-like structure, a plate structure, a hemi-sphere structure, a block structure, a spherical particle shape, or a sleeve shape. The three-dimensional porous bone implant material of the present disclosure can be used in an interbody fusion cage, an artificial vertebral body, an acetabular cup or an acetabular augment, a femoral stem and other products.

For example, the three-dimensional porous bone implant material is an interbody fusion cage, and the surface structure of the interbody fusion cage is a porous structure, such as a trabecular bone structure. The three-dimensional porous bone implant material can also be an artificial vertebral body, and the surface structure of the artificial vertebral body is a porous structure, such as a trabecular bone structure. For example, the three-dimensional porous bone implant material is an acetabular cup or an acetabular augment, and the surface structure of the acetabular cup or the acetabular augment is one or more of a porous structure(such as a trabecular bone structure), a titanium bead, a titanium powder, and a titanium wire. For another example, the three-dimensional porous bone implant material can also be a femoral stem, and the femoral proximal surface structure of the femur of the femoral stem is a porous structure.

In an embodiment of the present disclosure, the above-mentioned three-dimensional porous bone implant material is an interbody fusion cage, the height of the interbody fusion cage is preferably 4 to 15 mm, the diameter of the inscribed circle of the structural hole of the interbody fusion cage is preferably 4 to 10 mm, the diameter of the circumscribed circle of the interbody fusion cage is preferably 10 to 30 mm, and the included angle between the upper and lower end faces of the interbody fusion cage is preferably 0 to 15°.

In an another embodiment of the present disclosure, the described three-dimensional porous bone implant material is an artificial vertebral body, the height of the artificial vertebral body is preferably 15 to 200 mm, the diameter of the inscribed circle of a structural hole of the artificial vertebral body is preferably 4 to 10 mm, the diameter of the circumscribed circle of the artificial vertebral body is preferably 8 to 30 mm, and the included angle between upper and lower end faces of the artificial vertebral body is preferably 0 to 15°.

In an embodiment of the present disclosure, the three-dimensional porous bone implant material is an acetabular cup, the effective spherical outer diameter of the acetabular cup is preferably 36 to 72 mm, and the acetabular height of the acetabular cup is preferably 22 to 50 mm.

In an another embodiment of the present disclosure, the three-dimensional porous bone implant material is a femoral stem, the diameter of the stem body of the femoral stem is preferably Φ12 to Φ18 mm, and the CT value of the femoral stem is preferably 100 to 280 mm.

Beneficial effects of the present disclosure will be further described below with reference to the examples and comparative examples.

### Embodiment 1

An artificial vertebral body of Ti-6AI-4V is subjected to antibacterial treatment, the height of the artificial vertebral body is 50 mm, the diameter of the inscribed circle of the structural hole of the artificial vertebral body is 5 mm, the diameter of the circumscribed circle of the artificial vertebral body is 15 mm, the included angle between upper and lower end faces of the artificial vertebral body is 5°, the porosity thereof is 60%, and the average pore diameter thereof is 800 µm.

The artificial vertebral body is subjected to pickling, the volume ratio of the pickling solution is hydrofluoric acid with a concentration of 40%:nitric acid with a concentration of 68%:deionized water = 6:12:82; and the titanium material is pickled with the pickling solution for 15 minutes, then ultrasonically washed with deionized water for 10 minutes, and then sprayed, washed and dried so as to obtain a pretreated material.

The pretreated material is used as an anode, stainless steel is used as a cathode, the pretreated material and stainless steel are placed in the electrolyte for micro-arc oxidation of porous materials, an in-situ growth film layer having Zn ions and a biological activity is formed on the surface of the porous bone implant material, and the film layer has a thickness of 3 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; and the electrolyte also comprises zinc acetate with the concentration of 20 g/L, the process conditions of the micro-arc oxidation treatment are: an oxidation voltage of 700V, the output pulse frequency of the power source of 1200 Hz, the peak current being set to 600 A, and the oxidation time of 20 min. In the process of oxidation, the temperature of the electrolyte is controlled at about 10°C.

The artificial vertebral body having an in-situ growth film is ultrasonically washed with deionized water for 20min and then dried at 60°C so as to obtain the washed film.

The artificial vertebral body having a washed film is subjected to hydrothermal treatment, specifically the artificial vertebral body is placed into a high pressure reactor charged with a sodium hydroxide solution having a pH value of 10 to 12, and is subjected to hydrothermal treatment for 10 hours, wherein the solution temperature is 150°C, and the pressure is 10 MPa.

After the hydrothermal treatment, the artificial vertebral body is taken out, ultrasonically washed with deionized water for 10 min, then sprayed and washed with deionized water, and then air-dried in a cold air manner, so as to obtain a film layer having both antibacterial activity and biological activity on the surface of the artificial vertebral body.

### Embodiment 2

This embodiment differs from embodiment 1 in that:
The concentration of the zinc acetate in the electrolyte is 35 g/L, the process conditions of the micro-arc oxidation treatment are: an oxidation voltage of 600V, an output pulse frequency of a power source of 1000 Hz, a peak current being set to 500 A, and an oxidation time of 20 min. In the process of the oxidation, the temperature of the electrolyte is controlled at about 10°C.

### Embodiment 3

This embodiment differs from embodiment 1 in that:
The concentration of the zinc acetate in the electrolyte is 1 g/L, the process conditions of the micro-arc oxidation treatment are: an oxidation voltage of 800V, an output pulse frequency of a power source of 1500 Hz, a peak current being set to 800 A, and an oxidation time of 60 min. In the process of the oxidation, the temperature of the electrolyte is controlled at about 10°C.

### Embodiment 4

This embodiment differs from embodiment 1 in that:
The concentration of the zinc acetate in the electrolyte is 40 g/L, the process conditions of the micro-arc oxidation treatment are: an oxidation voltage of 600V, an output pulse frequency of a power source of 1000 Hz, a peak current being set to 500 A, and an oxidation time of 20 min. In the process of the oxidation, the temperature of the electrolyte is controlled at about 10°C.

### Embodiment 5

This embodiment differs from embodiment 1 in that:
The zinc acetate is replaced with zinc sulphate, the concentration of the zinc sulphate in the electrolyte is 20 g/L, the process conditions of the micro-arc oxidation treatment are: an oxidation voltage of 700V, an output pulse frequency of a power source of 1200 Hz, a peak current being set to 600 A; and the oxidation time of 20 min. In the process of the oxidation, the temperature of the electrolyte is controlled at about 10°C.

### Embodiment 6

This embodiment differs from embodiment 1 in that:
The electrolyte also contains copper acetate with a concentration of 10 g/L, the process conditions of the micro-arc oxidation treatment are: an oxidation voltage of 600V, an output pulse frequency of a power source of 1000 Hz, a peak current being set to 500 A, and an oxidation time of 20 min. In the process of the oxidation, the temperature of the electrolyte is controlled at about 10°C.

### Embodiment 7

This embodiment differs from embodiment 1 in that:
The electrolyte also contains copper acetate with a concentration of 20 g/L, the process conditions of the micro-arc oxidation treatment are: an oxidation voltage of 600V, an output pulse frequency of a power source of 1000 Hz, a peak current of 500 A, and an oxidation time of 20 min. In the process of the oxidation, the temperature of the electrolyte is controlled at about 10°C.

### Embodiment 8

This embodiment differs from embodiment 1 in that:
The zinc acetate is replaced with copper sulphate, the concentration of the copper sulphate in the electrolyte is 20 g/L, the process conditions of the micro-arc oxidation treatment are: an oxidation voltage of 700V, an output pulse frequency of a power source of 1200 Hz, a peak current being set to 600 A, and the oxidation time of 20 min. In the process of the oxidation, the temperature of the electrolyte is controlled at about 10°C.

### Embodiment 9

This embodiment differs from embodiment 1 in that:
The electrolyte also contains silver acetate with a concentration of 20 g/L, the process conditions of the micro-arc oxidation treatment are: an oxidation voltage of 600V, an output pulse frequency of a power source of 1000 Hz, a peak current being set to 500A, and an oxidation time of 20 min. In the process of the oxidation, the temperature of the electrolyte is controlled at about 10°C.

### Embodiment 10

This embodiment differs from embodiment 1 in that:
The electrolyte also contains silver acetate with a concentration of 40 g/L, the process conditions of the micro-arc oxidation treatment are: an oxidation voltage of 600V, an output pulse frequency of a power source of 1000 Hz, a peak current being set to 500A, and an oxidation time of 20 min. In the process of the oxidation, the temperature of the electrolyte is controlled at about 10°C.

### Embodiment 11

This embodiment differs from embodiment 1 in that:
The electrolyte also contains silver nitrate with a concentration of 20 g/L, the process conditions of the micro-arc oxidation treatment are: an oxidation voltage of 600V, an output pulse frequency of a power source of 1000 Hz, a peak current being set to 500A, and an oxidation time of 20 min. In the process of the oxidation, the temperature of the electrolyte is controlled at about 10°C.

### Embodiment 12

This embodiment differs from embodiment 1 in that:
The electrolyte also contains silver nitrate with a concentration of 35 g/L, the process conditions of the micro-arc oxidation treatment are: an oxidation voltage of 600V, an output pulse frequency of a power source of 1000 Hz, a peak current being set to 500A, and an oxidation time of 20 min. In the process of the oxidation, the temperature of the electrolyte is controlled at about 10°C.

### Embodiment 13

This embodiment differs from embodiment 1 in that:
The electrolyte also contains silver nitrate and copper sulphate, the concentration of the silver nitrate is 20 g/L, and the concentration of the copper sulphate is 10 g/L, the process conditions of the micro-arc oxidation treatment are: an oxidation voltage of 600V, an output pulse frequency of a power source of 1000 Hz, a peak current being set to 500 A, and an oxidation time of 20 min. In the process of the oxidation, the temperature of the electrolyte is controlled at about 10°C.

### Embodiment 14

This embodiment differs from embodiment 1 in that: an artificial vertebral body is subjected to pickling, the volume ratio of the pickling solution is hydrofluoric acid with a concentration of 40%:nitric acid with a concentration of 68%:deionized water = 5:15:80; and the titanium material is pickled with the pickling solution for 5 minutes, then ultrasonically washed with deionized water for 15 minutes, and then sprayed, washed and dried so as to obtain a pretreated material.

### Embodiment 15

This embodiment differs from embodiment 1 in that: an artificial vertebral body is subjected to pickling, the volume ratio of the pickling solution is hydrofluoric acid with a concentration of 40%:nitric acid with a concentration of 68%:deionized water = 6:10:84; and the titanium material is pickled with the pickling solution for 30 minutes, then ultrasonically washed with deionized water for 5 minutes, and then sprayed, washed and dried so as to obtain a pretreated material.

### Embodiment 16

This embodiment differs from embodiment 1 in that: an artificial vertebral body is subjected to pickling, the volume ratio of the pickling solution is hydrofluoric acid with a concentration of 40%:nitric acid with a concentration of 68%:deionized water = 3:20:77; and the titanium material is pickled with the pickling solution for 20 minutes, then ultrasonically washed with deionized water for 15 minutes, and then sprayed, washed and dried so as to obtain a pretreated material.

### Embodiment 17

This embodiment differs from embodiment 1 in that: an artificial vertebral body having a washed film is subjected to hydrothermal treatment, specifically the artificial vertebral body is placed into a high pressure reactor charged with a sodium hydroxide solution having a pH value of 8 to 10, and is subjected to thermal treatment for 32 hours, wherein the solution temperature is 120°C, and the pressure is 1 MPa.

### Embodiment 18

This embodiment differs from embodiment 1 in that: an artificial vertebral body having a washed film is subjected to hydrothermal treatment, specifically the artificial vertebral body is placed into a high pressure reactor charged with a sodium hydroxide solution having a pH value of 8 to 10, and is subjected to thermal treatment for 8 hours, wherein the solution temperature is 300°C, and the pressure is 15 MPa.

### Embodiment 19

This embodiment differs from embodiment 1 in that: an artificial vertebral body having a washed film is subjected to hydrothermal treatment, specifically the artificial vertebral body is placed into a high pressure reactor charged with a sodium hydroxide solution having a pH value of 11 to 12, and is subjected to thermal treatment for 20 hours, wherein the solution temperature is 250°C, and the pressure is 0.5 MPa.

### Embodiment 20

An artificial vertebral body of Ti-6AI-4V is subjected to anti-bacterial treatment, the height of the artificial vertebral body is 15mm, the diameter of the inscribed circle of the structural hole of the artificial vertebral body is 4mm, the diameter of the circumscribed circle of the artificial vertebral body is 8mm, the included angle between upper and lower end faces of the artificial vertebral body is 0°, the porosity thereof is 40%, and the average pore diameter thereof is 200 µm.

The artificial vertebral body is subjected to pickling, the volume ratio of the pickling solution is hydrofluoric acid with a concentration of 40%:nitric acid with a concentration of 68%:deionized water = 6:12:82; and the titanium material is pickled with the pickling solution for 2 minutes, then ultrasonically washed with deionized water for 5 minutes, and then sprayed, washed and dried so as to obtain a pretreated material.

The pretreated material is used as an anode, and stainless steel is used as a cathode, the pretreated material and stainless steel are placed in the electrolyte for micro-arc oxidation of porous materials, an in-situ growth film layer having Zn ions and a biological activity is formed on the surface of the porous bone implant material, and the film layer has a thickness of 5.3 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 20 g/L, the process conditions of the micro-arc oxidation treatment are: an oxidation voltage of 700V, the output pulse frequency of the power source of 1200 Hz, the peak current being set to 600 A, and the oxidation time of 15min. In the process of oxidation, the temperature of the electrolyte is controlled at about 10°C.

The artificial vertebral body having an in-situ growth film is ultrasonically washed with deionized water for 15min and then dried at 60°C so as to obtain the washed film.

An artificial vertebral body having a washed film is subjected to hydrothermal treatment, specifically the artificial vertebral body is placed into a high pressure reactor charged with a sodium hydroxide solution having a pH value of 10 to 12, and is subjected to thermal treatment for 10 hours, wherein the solution temperature is 150°C, and the pressure is 10 MPa.

After the hydrothermal treatment, the artificial vertebral body is taken out, ultrasonically washed with deionized water for 10 min, then sprayed and washed with deionized water, and then air-dried in a cold air manner, so as to obtain a film layer having both antibacterial activity and biological activity on the surface of the artificial vertebral body.

### Embodiment 21

An artificial vertebral body of Ti-6AI-4V is subjected to anti-bacterial treatment, the height of the artificial vertebral body is 200mm, the diameter of the inscribed circle of the structural hole of the artificial vertebral body is 10mm, the diameter of the circumscribed circle of the artificial vertebral body is 30mm, the included angle between upper and lower end faces of the artificial vertebral body is 15°, the porosity thereof is 90%, and the average pore diameter thereof is 1500 µm.

The artificial vertebral body is subjected to pickling, the volume ratio of the pickling solution is hydrofluoric acid with a concentration of 40%:nitric acid with a concentration of 68%:deionized water = 6:12:82; and the titanium material is pickled with the pickling solution for 20 minutes, then ultrasonically washed with deionized water for 15 minutes, and then sprayed, washed and dried so as to obtain a pretreated material.

The pre-treated material is used as an anode, and stainless steel is used as a cathode; the pre-treated material and stainless steel are placed in the electrolyte for micro-arc oxidation of porous materials, an in-situ growth film layer having Zn ions and a biological activity is formed on the surface of the porous bone implant material, and the film layer has a thickness of 2.7 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 35g/L, the process conditions of the micro-arc oxidation treatment are: an oxidation voltage of 1000V, the output pulse frequency of the power source of 1200 Hz, the peak current being set to 800A, and the oxidation time of 40min. In the precoess of oxidation, the temperature of the electrolyte is controlled at about 10°C.

The artificial vertebral body having an in-situ growth film is ultrasonically washed with deionized water for 20min and then dried at 60°C so as to obtain the washed film.

The artificial vertebral body having a washed film is subjected to hydrothermal treatment, specifically the artificial vertebral body is placed into a high pressure reactor charged with a sodium hydroxide solution having a pH value of 10 to 12, and is subjected to hydrothermal treatment for 10 hours, wherein the solution temperature is 150°C, and the pressure is 10 MPa.

After the hydrothermal treatment, the artificial vertebral body is taken out, ultrasonically washed with deionized water for 10 min, then sprayed and washed with deionized water, and then air-dried in a cold air manner, so as to obtain a film layer having both antibacterial activity and biological activity on the surface of the artificial vertebral body.

### Embodiment 22

This embodiment differs from embodiment 1 in that: an artificial vertebral body of Ti-6AI-4V is subjected to antibacterial treatment, the height of the artificial vertebral body is 100 mm, the diameter of the inscribed circle of the structural hole of the artificial vertebral body is 15 mm, the diameter of the circumscribed circle of the artificial vertebral body is 40 mm, the included angle of upper and lower end faces of the artificial vertebral body is 10°, the porosity thereof is 60%, and the average pore size thereof is 800 µm.

### Embodiment 23

This embodiment differs from embodiment 1 in that: the three-dimensional porous bone implant material is an interbody fusion cage, the height of the interbody fusion cage is 12mm, the diameter of the inscribed circle of the structural hole is 4mm, the diameter of the circumscribed circle of the interbody fusion cage is 20mm, the included angle between the upper and lower end faces of the interbody fusion cage is about 10°, the porosity thereof is 75%, and the average pore diameter thereof is 700 µm.

### Embodiment 24

An interbody fusion cage of Ti-6AI-4V is subjected to antibacterial treatment, the height of the interbody fusion cage is 15 mm, the diameter of the inscribed circle of a structural hole is 10 mm, the diameter of the circumscribed circle of the interbody fusion cage is 30 mm, the included angle between the upper and lower end faces of the interbody fusion cage is about 15°, the porosity thereof is 75%, and the average pore size thereof is 700 µm.

The intervertebral fusion cage is subjected to pickling, the volume ratio of the pickling solution is hydrofluoric acid with a concentration of 40%:nitric acid with a concentration of 68%:deionized water = 6:12:82; and the titanium material is pickled with the pickling solution for 10 minutes, then ultrasonically washed with deionized water for 10 minutes, and then sprayed, washed and dried so as to obtain a pretreated material.

The pre-treated material is used as an anode, and stainless steel is used as a cathode; the pre-treated material and stainless steel are placed in the electrolyte for micro-arc oxidation of porous materials, an in-situ growth film layer having Zn ions and a biological activity is formed on the surface of the porous bone implant material, and the film layer has a thickness of 5.8 µm, Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 20g/L, the process conditions of the micro-arc oxidation treatment are: an oxidation voltage of 700V, the output pulse frequency of the power source of 1200 Hz, the peak current being set to 600A, and the oxidation time of 20min. In the process of oxidation, the temperature of the electrolyte is controlled at about 10°C.

The intervertebral fusion cage having an in-situ growth film is ultrasonically washed with deionized water for 20min and then dried at 60°C so as to obtain the washed film.

An intervertebral fusion cage having a washed film is subjected to hydrothermal treatment, specifically the artificial vertebral body is placed into a high pressure reactor charged with a sodium hydroxide solution having a pH value of 10 to 12, and is subjected to thermal treatment for 10 hours, wherein the solution temperature is 150°C, and the pressure is 10 MPa.

After the hydrothermal treatment, the interbody fusion cage is taken out, ultrasonically washed with deionized water for 10 min, then sprayed and washed with deionized water, and then air-dried in a cold air manner, so as to obtain a film layer having both antibacterial activity and biological activity on the surface of the interbody fusion cage.

### Embodiment 25

An interbody fusion cage of Ti-6AI-4V is subjected to antibacterial treatment, the height of the interbody fusion cage is 4 mm, the diameter of the inscribed circle of the structural hole is 4 mm, the diameter of the circumscribed circle of the interbody fusion cage is 10 mm, the included angle between the upper and lower end faces of the interbody fusion cage is about 0°, the porosity thereof is 75%, and the average pore size thereof is 700 µm.

The intervertebral fusion cage is subjected to pickling, the volume ratio of the pickling solution is hydrofluoric acid with a concentration of 40%:nitric acid with a concentration of 68%:deionized water = 6:12:82; and the titanium material is pickled with the pickling solution for 2 minutes, then ultrasonically washed with deionized water for 10 minutes, and then sprayed, washed and dried so as to obtain a pretreated material.

The pretreated material is used as an anode, and stainless steel is used as a cathode; the pretreated material and stainless steel are placed in the electrolyte for micro-arc oxidation of porous materials, an in-situ growth film layer having Zn ions and a biological activity is formed on the surface of the porous bone implant material, and the film layer has a thickness of 7 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 20g/L, the process conditions of the micro-arc oxidation treatment are: an oxidation voltage of 400V, the output pulse frequency of the power source of 600Hz, the peak current being set to 300A, and the oxidation time of 5min. In the process of oxidation, the temperature of the electrolyte is controlled at about 10°C.

The intervertebral fusion cage having an in-situ growth film is ultrasonically washed with deionized water for 15min and then dried at 60°C so as to obtain the washed film.

An intervertebral fusion cage having a washed film is subjected to hydrothermal treatment, specifically the artificial vertebral body is placed into a high pressure reactor charged with a sodium hydroxide solution having a pH value of 10 to 12, and is subjected to thermal treatment for 2 hours, wherein the solution temperature is 150°C, and the pressure is 10 MPa.

After the hydrothermal treatment, the interbody fusion cage is taken out, ultrasonically washed with deionized water for 10 min, then sprayed and washed with deionized water, and then air-dried in a cold air manner, so as to obtain a film layer having both antibacterial activity and biological activity on the surface of the interbody fusion cage.

### Embodiment 26

This embodiment differs from embodiment 1 in that: the three-dimensional porous bone implant material is an interbody fusion cage of Ti-6AI-4V, the height of the interbody fusion cage is 20mm, the diameter of the inscribed circle of the structural hole is 10mm, the diameter of the circumscribed circle of the interbody fusion cage is 40mm, the included angle between the upper and lower end faces of the interbody fusion cage is about 10°C, the porosity thereof is 75%, and the average pore diameter thereof is 700 µm.

### Embodiment 27

This embodiment differs from embodiment 1 in that: the three-dimensional porous bone implant material is an acetabular cup, the acetabular cup has the effective spherical outer diameter of 54 mm and the acetabular height of 26 mm, the porosity of 75%, and the average pore size of 600 µm.

### Embodiment 28

An acetabular cup of Ti-6AI-4V is subjected to antibacterial treatment, the acetabular cup has the effective spherical outer diameter of 36 mm and the acetabular height of 22 mm, the porosity of 75%, and the average pore size of 600 µm.

The acetabular cup is subjected to pickling, the volume ratio of the pickling solution is hydrofluoric acid with a concentration of 40%:nitric acid with a concentration of 68%:deionized water = 6:12:82; and the titanium material is pickled with the pickling solution for 15 minutes, then ultrasonically washed with deionized water for 15 minutes, and then sprayed, washed and dried so as to obtain a pretreated material.

The pretreated material is used as an anode, and stainless steel is used as a cathode; the pretreated material and stainless steel are placed in the electrolyte for micro-arc oxidation of porous materials, an in-situ growth film layer having Zn ions and a biological activity is formed on the surface of the porous bone implant material, and the film layer has a thickness of 4.2 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 20 g/L, the process conditions of the micro-arc oxidation treatment are: an oxidation voltage of 700V, the output pulse frequency of the power source of 1200 Hz, the peak current being set to 600 A, and the oxidation time of 20min. In the process of oxidation, the temperature of the electrolyte is controlled at about 10°C.

The acetabular cup having an in-situ growth film is ultrasonically washed with deionized water for 20min and then dried at 60°C so as to obtain the washed film.

The acetabular cup having a washed film is subjected to hydrothermal treatment, specifically the artificial vertebral body is placed into a high pressure reactor charged with a sodium hydroxide solution having a pH value of 10 to 12, and is subjected to thermal treatment for 10 hours, wherein the solution temperature is 150°C, and the pressure is 10 MPa.

After the hydrothermal treatment, the acetabular cup is taken out, ultrasonically washed with deionized water for 10 min, then sprayed and washed with deionized water, and then air-dried in a cold air manner, so as to obtain a film layer having both antibacterial activity and biological activity on the surface of the acetabular cup.

### Embodiment 29

An acetabular cup of Ti-6AI-4V is subjected to antibacterial treatment, the acetabular cup has the effective spherical outer diameter of 72mm and the acetabular height of 50mm, the porosity of 75%, and the average pore size of 600 µm.

The acetabular cup is subjected to pickling, the volume ratio of the pickling solution is hydrofluoric acid with a concentration of 40%:nitric acid with a concentration of 68%:deionized water = 6:12:82; and the titanium material is pickled with the pickling solution for 30 minutes, then ultrasonically washed with deionized water for 10 minutes, and then sprayed, washed and dried so as to obtain a pretreated material;

The pretreated material is used as an anode, and stainless steel is used as a cathode; the pretreated material and stainless steel are placed in the electrolyte for micro-arc oxidation of porous materials, an in-situ growth film layer having Zn ions and a biological activity is formed on the surface of the porous bone implant material, and the film layer has a thickness of 2 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 35g/L, the process conditions of the micro-arc oxidation treatment are: an oxidation voltage of 1200V, the output pulse frequency of the power source of 1500Hz, the peak current being set to 800A, and the oxidation time of 60min. In the process of oxidation, the temperature of the electrolyte is controlled at about 10°C.

The acetabular cup having an in-situ growth film is ultrasonically washed with deionized water for 20min and then dried at 60°C so as to obtain the washed film.

The acetabular cup having a washed film is subjected to hydrothermal treatment, specifically the artificial vertebral body is placed into a high pressure reactor charged with a sodium hydroxide solution having a pH value of 10 to 12, and is subjected to thermal treatment for 32 hours, wherein the solution temperature is 150°C, and the pressure is 10 MPa.

After the hydrothermal treatment, the acetabular cup is taken out, ultrasonically washed with deionized water for 10 min, then sprayed and washed with deionized water, and then air-dried in a cold air manner, so as to obtain a film layer having both antibacterial activity and biological activity on the surface of the acetabular cup.

### Embodiment 30

This embodiment differs from embodiment 1 in that: the three-dimensional porous bone implant material is an acetabular cup of Ti-6AI-4V. The acetabular cup has the effective spherical outer diameter of 30mm and the acetabular height of 20mm, the porosity of 75%, and the average pore size of 700 µm.

### Embodiment 31

This embodiment differs from embodiment 1 in that: the three-dimensional porous bone implant material is a femoral stem, the femoral stem has the stem diameter of Φ15 mm and the CT value of 170 mm. The porous structure has the porosity of 60% and the average pore size of 600 µm.

### Embodiment 32

A femoral stem of Ti-6AI-4V is subjected to antibacterial treatment, and the femoral stem has the stem diameter of Φ12 mm and the CT value of 100 mm. The porous structure has the porosity of 60% and the average pore size of 600 µm.

The femoral stem is subjected to pickling, the volume ratio of the pickling solution is hydrofluoric acid with a concentration of 40%:nitric acid with a concentration of 68%:deionized water = 6:12:82; and the titanium material is pickled with the pickling solution for 15 minutes, then ultrasonically washed with deionized water for 10 minutes, and then sprayed, washed and dried so as to obtain a pretreated material.

The pretreated material is used as an anode, and stainless steel is used as a cathode; the pretreated material and stainless steel are placed in the electrolyte for micro-arc oxidation of porous materials, an in-situ growth film layer having Zn ions and a biological activity is formed on the surface of the porous bone implant material, and the film layer has a thickness of 4.9 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 20g/L, the process conditions of the micro-arc oxidation treatment are: an oxidation voltage of 700V, the output pulse frequency of the power source of 1200Hz, the peak current being set to 600A, and the oxidation time of 10min. In the process of oxidation, the temperature of the electrolyte is controlled at about 10°C.

The femoral stem having an in-situ growth film is ultrasonically washed with deionized water for 20min and then dried at 60°C so as to obtain the washed film.

The femoral stem having a washed film is subjected to hydrothermal treatment, specifically the artificial vertebral body is placed into a high pressure reactor charged with a sodium hydroxide solution having a pH value of 10 to 12, and is subjected to thermal treatment for 10 hours, wherein the solution temperature is 150°C, and the pressure is 10 MPa.

After the hydrothermal treatment, the femoral stem is taken out, ultrasonically washed with deionized water for 10 min, then sprayed and washed with deionized water, and then air-dried in a cold air manner, so as to obtain a film layer having both antibacterial activity and biological activity on the surface of the femoral stem.

### Embodiment 33

A femoral stem of Ti-6AI-4V is subjected to antibacterial treatment, wherein the femoral stem has the stem diameter of Φ18 mm and the CT value of 280mm. The porous structure has the porosity of 60% and the average pore size of 600 µm.

The femoral stem is subjected to pickling, the volume ratio of the pickling solution is hydrofluoric acid with a concentration of 40%:nitric acid with a concentration of 68%: deionized water = 6:12:82; and the titanium material is pickled with the pickling solution for 15 minutes, then ultrasonically washed with deionized water for 10 minutes, and then sprayed, washed and dried so as to obtain a pretreated material;

The pretreated material is used as an anode, and stainless steel is used as a cathode; the pretreated material and stainless steel are placed in the electrolyte for micro-arc oxidation of porous materials, an in-situ growth film layer having Zn ions and a biological activity is formed on the surface of the porous bone implant material, and the film layer has a thickness of 5.1 µm. Wherein the bioactive elements in the electrolyte are calcium and phosphorus; in the electrolyte, the concentration of calcium element is 0.02 mol/L, and the concentration of phosphorus element is 0.045 mol/L; the calcium source is calcium acetate, and the phosphorus source is sodium dihydrogen phosphate; EDTA-2Na is added in the electrolyte as a complexing agent, the concentration of EDTA in the electrolyte is recorded as 0.02 mol/L; sodium hydroxide or potassium hydroxide is added in the electrolyte to adjust the pH value of the electrolyte to about 12; the electrolyte also comprises zinc acetate with the concentration of 20g/L, the process conditions of the micro-arc oxidation treatment are: an oxidation voltage of 900V, the output pulse frequency of the power source of 1200Hz, the peak current being set to 750A, and the oxidation time of 20min. In the process of oxidation, the temperature of the electrolyte is controlled at about 10°C.

The femoral stem having an in-situ growth film is ultrasonically washed with deionized water for 20min and then dried at 60°C so as to obtain the washed film.

The femoral stem having a washed film is subjected to hydrothermal treatment, specifically the artificial vertebral body is placed into a high pressure reactor charged with a sodium hydroxide solution having a pH value of 10 to 12, and is subjected to thermal treatment for 10 hours, wherein the solution temperature is 150°C, and the pressure is 10 MPa.

After the hydrothermal treatment, the femoral stem is taken out, ultrasonically washed with deionized water for 10 min, then sprayed and washed with deionized water, and then air-dried in a cold air manner, so as to obtain a film layer having both antibacterial activity and biological activity on the surface of the femoral stem.

### Embodiment 34

This embodiment differs from embodiment 1 in that: the three-dimensional porous bone implant material is a femoral stem of Ti-6AI-4V, the femoral stem has the stem diameter of 10 mm and the CT value of 120 mm. The porous structure has the porosity of 60% and the average pore size of 600 µm.

### Comparative Embodiment 1

This embodiment differs from embodiment 1 in that: the artificial vertebral bodies is directly subjected to micro-arc oxidation treatment without activating treatment.

### Comparative Embodiment 2

This embodiment differs from embodiment 1 in that: after the micro-arc oxidation treatment is completed, only the artificial vertebral body having an in-situ growth layer is ultrasonically washed with deionized water for 20min and then dried at 60°C so as to obtain a cleaning film, without subsequent hydrothermal treatment.

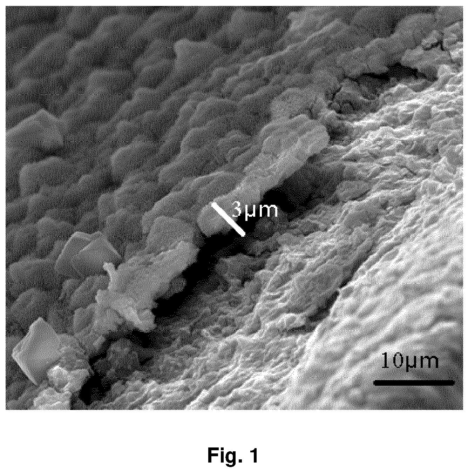

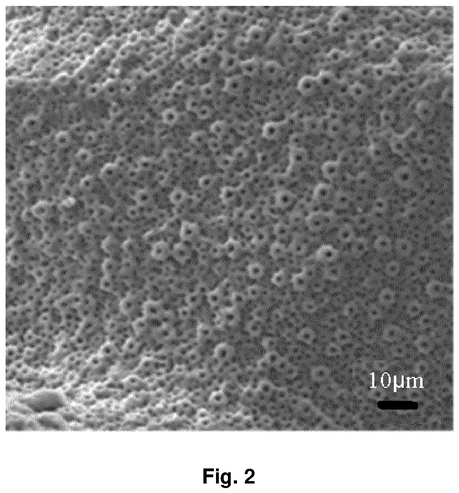

The thickness and pore size of the in-situ growth film layer of the antibacterial three-dimensional porous bone implant materials formed in Embodiments 1 to 34 and Comparative Embodiments 1 and 2 are detected by scanning electron microscope cross-section method, the porosity of the film layer is tested by scanning electron microscope photographs in combination with an image pro plus 6.0 software. The scanning electron microscope photographs of Embodiment 1 are shown in Figs. 1 and 2, in which Fig. 1 shows the clear thickness of the film layer, and Fig. 2 shows that there are micropores on the surface of the film layer. Element compositions and antibacterial ion contents of the film surfaces of the implant surfaces of Embodiments 1 to 34 and Comparative Embodiments 1 to 2 are measured by EDS spectroscopy and are recorded in Table 1.

**Table 1**

| | Film thickness /µm | Mean pore size/µm | Porosity /% | Calcium, phosphorus and antibacterial elements on the surface film layer and their respective atomic percentages | | | | |
|---|---|---|---|---|---|---|---|---|
| | | | | Calcium | Phosphorus | Zinc | Copper | Silver |
| Embodiment 1 | 3.0 | 0.95 | 16.4 | 8.32 | 2.87 | 1.92 | - | - |
| Embodiment 2 | 3.7 | 1.16 | 19.2 | 9.01 | 2.79 | 2.03 | - | - |
| Embodiment 3 | 3.5 | 0.87 | 16.5 | 8.85 | 2.38 | 0.57 | - | - |
| Embodiment 4 | 3.4 | 0.92 | 15.9 | 8.97 | 2.45 | 1.93 | - | - |
| Embodiment 5 | 3.5 | 1.03 | 17.2 | 8.87 | 2.19 | 1.82 | - | - |
| Embodiment 6 | 3.5 | 0.92 | 16.8 | 8.48 | 2.90 | 1.79 | 0.76 | - |
| Embodiment 7 | 3.9 | 1.01 | 16.9 | 8.41 | 2.80 | 1.62 | 1.45 | - |
| Embodiment 8 | 3.9 | 1.20 | 17.4 | 8.58 | 2.73 | - | 1.82 | - |
| Embodiment 9 | 3.4 | 1.16 | 16.8 | 8.19 | 2.63 | 1.78 | - | 1.31 |
| Embodiment 10 | 3.6 | 1.26 | 18.1 | 8.42 | 2.59 | 1.73 | - | 1.29 |
| Embodiment 11 | 3.2 | 0.99 | 17.2 | 8.91 | 2.31 | 1.65 | - | 1.26 |
| Embodiment 12 | 3.3 | 1.01 | 16.2 | 8.77 | 2.60 | 1.60 | - | 1.28 |
| Embodiment 13 | 3.8 | 1.40 | 19.1 | 8.99 | 2.93 | 1.58 | 0.85 | 1.24 |
| Embodiment 14 | 3.3 | 1.05 | 15.9 | 8.62 | 2.91 | 1.72 | - | - |
| Embodiment 15 | 3.1 | 1.15 | 16.3 | 8.71 | 2.86 | 1.69 | - | - |
| Embodiment 16 | 3.7 | 0.95 | 15.3 | 8.95 | 2.38 | 1.29 | - | - |
| Embodiment 17 | 3.6 | 1.05 | 17.3 | 8.22 | 2.77 | 1.64 | - | - |
| Embodiment 18 | 3.5 | 1.02 | 16.5 | 8.52 | 2.60 | 1.59 | - | - |
| Embodiment 19 | 3.4 | 1.16 | 16.7 | 8.72 | 2.81 | 1.39 | - | - |
| Embodiment 20 | 5.3 | 1.24 | 19.6 | 9.70 | 3.21 | 2.01 | - | - |
| Embodiment 21 | 2.7 | 1.10 | 16.2 | 8.97 | 2.65 | 1.78 | - | - |
| Embodiment 22 | 1.0 | 0.32 | 10.8 | 4.93 | 0.71 | 1.32 | - | - |
| Embodiment 23 | 4.2 | 1.35 | 19.4 | 9.37 | 3.07 | 2.12 | - | - |
| Embodiment 24 | 5.8 | 1.41 | 20.2 | 10.31 | 4.32 | 1.98 | - | - |
| Embodiment 25 | 7.0 | 1.32 | 18.7 | 10.71 | 4.28 | 1.83 | - | - |
| Embodiment 26 | 1.2 | 0.67 | 10.9 | 6.98 | 3.98 | 1.17 | - | - |
| Embodiment 27 | 2.6 | 0.85 | 13.4 | 8.27 | 2.57 | 1.49 | - | - |
| Embodiment 28 | 4.2 | 1.27 | 20.1 | 11.25 | 4.98 | 1.97 | - | - |
| Embodiment 29 | 2.0 | 0.92 | 17.8 | 10.10 | 4.21 | 1.62 | - | - |
| Embodiment 30 | 8.0 | 0.78 | 12.6 | 5.72 | 2.61 | 1.08 | - | - |
| Embodiment 31 | 5.2 | 1.55 | 22.4 | 10.37 | 4.07 | 2.03 | - | - |
| Embodiment 32 | 4.9 | 1.43 | 20.8 | 11.31 | 3.99 | 1.80 | - | - |
| Embodiment 33 | 5.1 | 1.20 | 18.2 | 10.92 | 3.71 | 1.72 | - | - |
| Embodiment 34 | 10.0 | 0.91 | 10.9 | 9.29 | 2.57 | 1.22 | - | - |
| Comparative Embodiment 1 | 2.6 | 0.75 | 11.4 | 5.32 | 1.87 | 0.98 | - | - |
| Comparative Embodiment 2 | 3.3 | 1.05 | 17.1 | 8.62 | 2.97 | 0.94 | - | - |

The antibacterial effects of the antibacterial three-dimensional porous bone implant materials prepared in the examples and comparative examples of the present invention is tested by a filming sticking method. Taking the antibacterial rates of Escherichia coli and Staphylococcus aureus as an example, and the specific method is as follows: cultivating Escherichia coli and Staphylococcus aureus strain on a culture medium for 24 h at 37°C for later use; inoculating the bacteria for two generations, adjusting the concentration of the bacterial solution to a certain value, taking out the implant and placing same into a culture dish, taking out the bacterial suspension, dropwise adding same onto the surface of the sample, after culturing for a certain period of time, taking out the sample and eluting same, dropping the eluate onto the culture medium plate uniformly, uniformly spreading the eluate, plate counting same after 24 h at 37°C, counting the average of colonies, and repeating the experiment three times. The antibacterial rate is calculated as: [(the number of control colonies in the control group - number of example colonies in the embodiment group)/ the number of control colonies in the control group] x 100%. The results of the characterization are shown in Table 2.

**Table 2**

| | Inhibition rate of Staphylococcus aureus (%) | Inhibition rate of Escherichia coli (%) |
|---|---|---|
| Embodiment 1 | 82.3 | 90.1 |
| Embodiment 2 | 83.2 | 91.7 |
| Embodiment 3 | 60.6 | 70.3 |
| Embodiment 4 | 81.3 | 89.1 |
| Embodiment 5 | 81.3 | 89.6 |
| Embodiment 6 | 83.7 | 91.9 |
| Embodiment 7 | 84.8 | 90.2 |
| Embodiment 8 | 81.7 | 90.8 |
| Embodiment 9 | 89.8 | 96.8 |
| Embodiment 10 | 88.6 | 85.2 |
| Embodiment 11 | 88.9 | 93.1 |
| Embodiment 12 | 89.6 | 96.2 |
| Embodiment 13 | 95.8 | 99.8 |
| Embodiment 14 | 78.7 | 85.6 |
| Embodiment 15 | 76.4 | 82.4 |
| Embodiment 16 | 70.1 | 78.6 |
| Embodiment 17 | 77.3 | 81.2 |
| Embodiment 18 | 75.2 | 80.9 |
| Embodiment 19 | 71.7 | 78.3 |
| Embodiment 20 | 84.9 | 93.2 |
| Embodiment 21 | 83.2 | 91.1 |
| Embodiment 22 | 70.9 | 80.7 |
| Embodiment 23 | 80.5 | 91.7 |
| Embodiment 24 | 85.2 | 93.7 |
| Embodiment 25 | 82.1 | 92.1 |
| Embodiment 26 | 72.1 | 80.3 |
| Embodiment 27 | 79.9 | 89.2 |
| Embodiment 28 | 84.2 | 93.6 |
| Embodiment 29 | 83.9 | 92.9 |
| Embodiment 30 | 72.1 | 80.3 |
| Embodiment 31 | 81.6 | 91.6 |
| Embodiment 32 | 80.6 | 89.9 |
| Embodiment 33 | 79.9 | 88.7 |
| Embodiment 34 | 71.2 | 80.2 |
| Comparative Embodiment 1 | 69.9 | 76.1 |
| Comparative Embodiment 2 | 62.7 | 72.4 |

From the above description, it can be determined that the above examples of the present invention achieve the following technical effects: by means of technologies such as surface activation and hydrothermal treatment, the porous titanium implant is subjected to surface treatment, and the obtained film layer has obvious inhibiting and bactericidal effects on Staphylococcus aureus, Escherichia coli and the like, greatly improving the antibacterial performance of the porous titanium bone implant material, and having important clinical significance for reducing the infection of the titanium implant.

The above description is only the preferred examples of the present invention, and is not intended to limit the present invention. For those skilled in the art, the present invention may have various modifications and variations. Any modifications, equivalent replacements, improvements and the like made within the spirit and principle of the present invention shall belong to the protection scope of the present invention.

## Claims

1. An antibacterial three-dimensional porous bone implant material, the antibacterial three-dimensional porous bone implant material comprises:
a three-dimensional porous bone implant material; and
an in-situ growth film layer in-situ growing on the surface of the three-dimensional porous bone implant material, wherein the in-situ growth film layer comprises a functional substance and an antibacterial substance, and the antibacterial substance comprises any one or more of zinc ions, copper ions or silver ions.

2. The antibacterial three-dimensional porous bone implant material according to claim 1, wherein the in-situ growth film layer has a thickness of 0.1 to 20 µm, preferably 2 to 7 µm.

3. The antibacterial three-dimensional porous bone implant material according to claim 1, wherein the surface of the in-situ growth film layer comprises micro pores with a pore diameter of 0.1 to 5 µm, and preferably the in-situ growth film layer has a porosity of 2 to 30%.

4. The antibacterial three-dimensional porous bone implant material according to claim 1, wherein in the in-situ growth film layer, the content of the zinc ions is 0.5 to 3 at.%, and/or the content of the copper ions is 0.5 to 3 at.%, and/or the content of the silver ions is 0.01 to 2 at.%.

5. The antibacterial three-dimensional porous bone implant material according to claim 1, wherein the surface on which the three-dimensional porous bone implant material is in contact with a host bone tissue is porous structure titanium or porous titanium alloy.

6. The antibacterial three-dimensional porous bone implant material according to claim 5, wherein the porous structure titanium is one of pure titanium, Ti-6AI-4V, Ti-6AI-17Nb, Ti-13Nb-13Zr or Ti-5Zr-3Mo-15Nb, and the porous titanium alloy is one of Ti-6AI-4V, Ti-6AI-17Nb, Ti-13Nb-13Zr or Ti-5Zr-3Mo-15Nb.

7. The antibacterial three-dimensional porous bone implant material according to claim 1, wherein the three-dimensional porous bone implant material is in a fully porous structure or a mixed structure of porous and solid structures.

8. The antibacterial three-dimensional porous bone implant material according to claim 7, wherein the three-dimensional porous bone implant material has a porosity of 40 to 90% and a pore diameter of 200 to 1500 µm.

9. The antibacterial three-dimensional porous bone implant material according to claim 7, wherein the porous structure of the three-dimensional porous bone implant material is one or more of a random pore structure, a cubic structure, a hexagonal prism structure, a diamond structure, a rhombic dodecahedron structure, a truncated octahedron structure, a titanium bead sintering structure, and a titanium wire sintering structure.

10. The antibacterial three-dimensional porous bone implant material according to claim 1, wherein the three-dimensional porous bone implant material is in a columnar structure, a columnar-like structure, a plate structure, a hemisphere structure, a block structure, a spherical particle shape, a conical or a sleeve shape.

11. A preparation method for an antibacterial three-dimensional porous bone implant material, comprising:
performing surface activation treatment on the three-dimensional porous bone implant material, so as to obtain a pretreated material;
performing micro-arc oxidation treatment on the pretreated material, so as to obtain an in-situ growth film layer having antibacterial ions formed on the surface of the pre-treated material, wherein an electrolyte used in the micro-arc oxidation treatment comprises antibacterial ions and a functional substance, and the antibacterial ions are selected from any one or more of zinc ions, copper ions and silver ions; and
performing hydrothermal treatment on the in-situ growth film layer, so as to obtain the antibacterial three-dimensional porous bone implant material.

12. The preparation method according to claim 11, wherein the process of the surface activation treatment comprises:
pickling the three-dimensional porous bone implant material, wherein the pickling solution used in the pickling comprises hydrofluoric acid, nitric acid and water, and is metered with the hydrofluoric acid having a concentration of 40%, the nitric acid having a concentration of 68% and the water, the volume ratio of the hydrofluoric acid, the nitric acid and the water is (5-7): (10-15): (80-85), and the pickling time is preferably 2-30 minutes;
after the pickling is finished, washing the three-dimensional porous bone implant material with deionized water, so as to obtain the pretreated material; and
preferably, the washing comprises first ultrasonic washing for 5 to 15 minutes, and after the ultrasonic washing is finished, spraying, washing and then drying the three-dimensional porous bone implant material.

13. The preparation method according to claim 11, wherein in the electrolyte, the zinc ions are derived from zinc acetate or zinc sulfate, and preferably the concentration of the zinc acetate is 1 to 35 g/L or the concentration of the zinc sulfate is 1 to 20 g/L; the copper ions are derived from copper acetate or copper sulfate, and preferably the concentration of the copper acetate is 1 to 20 g/L or the concentration of the copper sulfate is 1 to 20 g/L; and the silver ions are derived from silver acetate or silver nitrate, and preferably the concentration of the silver acetate is 0.01 to 40 g/L or the concentration of the silver nitrate is 0.01 to 35 g/L.

14. The preparation method according to claim 1, wherein the oxidation voltage of the micro-arc oxidation treatment is between 400 and 1200 V, the output pulse frequency of the power source is between 600 and 1500 Hz, the peak current is set to be between 300 and 800 A, the oxidation time is between 5 and 60 min, and the temperature of the electrolyte is preferably controlled to be lower than 10°C in the process of the micro-arc oxidation treatment.

15. The preparation method according to claim 11, wherein the process of the hydrothermal treatment comprises:
step A, washing the three-dimensional porous bone implant material having the in-situ growth film with deionized water, so as to obtain a washed film;
step B, placing the three-dimensional porous bone implant material having the washed film in an alkaline solution and treating the material in an environment of 120-200°C and 0.5-15 MPa, so as to obtain a hydrothermal treatment film, wherein the alkaline solution is preferably a sodium hydroxide aqueous solution or a potassium hydroxide aqueous solution having a pH value of 8-12, and the treatment time is preferably 8-32 hours; and
step C, washing the three-dimensional porous bone implant material having the hydrothermal treatment film with deionized water so as to obtain the antibacterial three-dimensional porous bone implant material,
preferably, the washing in the step A and the step C adopts ultrasonic vibration treatment.

16. The preparation method according to claim 11, wherein the surface on which the three-dimensional porous bone implant material is in contact with the host bone tissue is porous structure titanium or porous titanium alloy; and preferably, the preparation method further comprises a step of preparing the porous bone implant material by using one or more of a powder metallurgy method, a foaming method, a fiber sintering method, a plasma spraying method, a laser drilling method, laser stereoforming, selective laser sintering/melting, and electron beam melting technologies.

17. The preparation method according to claim 16, wherein the porous structure titanium is one of pure titanium, Ti-6AI-4V, Ti-6AI-17Nb, Ti-13Nb-13Zr or Ti-5Zr-3Mo-15Nb, and the porous titanium alloy is one of Ti-6AI-4V, Ti-6AI-17Nb, Ti-13Nb-13Zr or Ti-5Zr-3Mo-15Nb;
preferably, the three-dimensional porous bone implant material having a structure pore which is a columnar structure, a columnar-like structure, a plate structure, a hemisphere structure, a block structure, a spherical particle shape, a conical or a sleeve shape;
preferably, the three-dimensional porous bone implant material is in a fully porous structure or a mixed structure of porous and a solid structures; more preferably, the three-dimensional porous bone implant material has a porosity of 40 to 90% and a pore diameter of 200 to 1500 µm; further preferably, the porous structure of the three-dimensional porous bone implant material is in one or more of a random pore structure, a cubic structure, a hexagonal prism structure, a diamond structure, a rhombic dodecahedron structure, a truncated octahedron structure, a titanium bead sintering structure, and a titanium wire sintering structure.

18. The preparation method according to any one of claims 11 to 17, wherein the in-situ growth film layer has a thickness of 0.1 to 20 µm, the surface of the in-situ growth film layer preferably comprises micropores with a pore diameter of 0.1 to 5 µm, and further preferably the in-situ growth film layer has a porosity of 2 to 30%.

19. The preparation method according to any one of claims 11 to 17, wherein the three-dimensional porous bone implant material is an acetabular cup or an acetabular augment, and the surface structure of the acetabular cup or the acetabular augment is one or more of a porous structure, a titanium bead, a titanium powder, and a titanium wire.

20. The preparation method according to any one of claims 11 to 17, wherein the three-dimensional porous bone implant material is an interbody fusion cage, and the surface structure of the interbody fusion cage is a porous structure

21. The preparation method according to any one of claims 11 to 17, wherein the three-dimensional porous bone implant material is an artificial vertebral body, and the surface structure of the artificial vertebral body is a porous structure.

22. The preparation method according to any one of claims 11 to 17, wherein the three-dimensional porous bone implant material is a femoral stem, and the proximal surface structure of the femur of the femoral stem is a porous structure.
